# EUROPÄISCHE PATENTSCHRIFT

(11) **EP 2 329 793 B1**
(45) Veröffentlichungstag und Bekanntmachung des Hinweises auf die Patenterteilung: **22.04.2015**
(21) Anmeldenummer: 10193566.6
(22) Anmeldetag: 02.12.2010
(51) Int. Cl.: A61D 19/02, A61B 17/43

(54) **Kathetervorrichtung zur künstlichen Befruchtung**
Catheter for artificial insemination
Cathéter pour insémination artificielle

(30) Priorität: 02.12.2009 DE 102009047423
(43) Veröffentlichungstag der Anmeldung: 08.06.2011
(73) Patentinhaber: German Genetics International GmbH, 49661 Cloppenburg (DE)
(72) Erfinder: Großfeld, Rudolf, 31535 Neustadt (DE); Rath, Detlef, 31535 Neustadt (DE)
(74) Vertreter: Taruttis, Stefan Georg

(56) Entgegenhaltungen:
- EP-A1- 1 177 776
- WO-A1-97/14365
- WO-A2-02/35982
- US-A- 5 624 399

## Beschreibung

Die vorliegende Erfindung betrifft eine Kathetervorrichtung, insbesondere eine Kathetervorrichtung zur unchirurgischen Übertragung von Gameten in den Eileiter (GIFT) von nicht-menschlichen Säugetieren, sowie die Verwendung der Kathetervorrichtung für die nicht-chirurgische Übertragung von Flüssigkeiten in einen oder aus einem Eileiter weiblicher Säugetiere, insbesondere für die nicht-chirurgische Gametenübertragung in einen Eileiter, z.B. für die endoskopische Fertilisierung weiblicher Säugetiere, insbesondere nicht-menschlicher Säugetiere, vorzugsweise von Huftieren, z.B. Pferden, bevorzugt von Paarhufern, z.B. von Rindern, Schweinen, Schafen und Ziegen. Gattungsgemäße Kathetervorrichtungen zur nicht-chirurgischen Besamung sind so gestaltet, dass ein starrer oder flexibler Schlauch bis in die Scheide oder maximal bis ins tiefe Uterushorn angeordnet werden kann, um dort Spermatozoen oder Embryonen einzubringen. Für die Übertragung von Oozyten ist deren Positionierung im Eileiter erforderlich. Dies ist bislang ausschließlich chirurgisch oder laparoskopisch durch die Bauchdecke und Bauchhöhle oder parauterin möglich.

Die erfindungsgemäße Kathetervorrichtung weist eine Gestalt auf, die für ein Verfahren bzw. für die Verwendung zur Entnahme oder zur Einbringung von Flüssigkeiten in den Eileiter, insbesondere von Flüssigkeiten, die Spermatozoen und/oder Oozyten enthalten, in den Eileiter insbesondere eines Rindes geeignet ist, sodass diese Vorrichtung insbesondere zur Verwendung als Katheter bzw. in einem Verfahren zum Spermientransfer direkt in den Eileiter (intratubaler Transfer von Spermien), bevorzugt für den Transfer geschlechtsspezifisch sortierter Spermatozoen beim Rind geeignet ist. Die Kathetervorrichtung ist ausschließlich durch natürliche Körperöffnungen eines weiblichen Säugetiers anordnbar und daher für ein nicht-chirurgisches Verfahren, bzw. für ein nichtmedizinisches Verfahren geeignet. Bei Verwendung der erfindungsgemäßen Vorrichtung, beispielsweise in einem nicht-chirurgischen Verfahren zum Gametentransfer, kann eine Spermatozoen und/oder Oozyten enthaltene Flüssigkeit direkt in den Eileiter eingebracht werden, insbesondere in den Abschnitt des Eileiters, der an die utero-tubale Verbindung angrenzt, und/oder in einen Abschnitt des Eileiters zwischen Fimbrientrichter und uterotubaler Verbindung. Die Verwendung der Kathetervorrichtung stellt keinen chirurgischen Eingriff beim Tier dar, weil sie ausschließlich durch natürliche Öffnungen des weiblichen Genitaltrakts einführbar ist, d.h. unblutig bzw. ohne eine Gewebsverletzung zu verursachen.

Entsprechend ist auch das erfindungsgemäße Verfahren zum Transfer von Flüssigkeiten in den oder aus einem Eileiter, insbesondere bei nicht-menschlichen Säugetieren, nicht medizinisch, sondern kann von einem Techniker durchgeführt werden und dient insbesondere zur nicht-medizinischen Probeentnahme oder zur künstlichen Fertilisierung, z.B. durch intrabubalen Gametentransfer. Das erfindungsgemäße Verfahren zum Transfer von Flüssigkeiten in einen Eileiter ist daher ein nicht medizinisches Verfahren und insbesondere keine Heilbehandlung, sondern ein Verfahren zur Etablierung einer Trächtigkeit.

### Stand der Technik

Die US 4,654,025 beschreibt einen Besamungskatheter, der insbesondere für die künstliche Besamung kleiner Raubtiere dadurch geeignet ist, dass er über die Länge zwei aufblasbare Ballons aufweist, mit denen in Entsprechung zum natürlichen Paarungsakt der weibliche Genitaltrakt abgedichtet werden kann.

Die DE 19606925 B4 beschreibt einen Katheter für die künstliche Besamung von Elefanten, der an seiner Spitze zwei separat aufblasbare Ballons aufweist.

Die EP 0214043 beschreibt einen Katheter zur chirurgischen Besamung, der transperitoneal anzuordnen ist.

Die DE 29703451 U1 beschreibt einen Besamungskatheter für Schweine, der auf seiner Außenseite eine Schraubenwendel aufweist, die vorzugsweise durch nebeneinander angeordnete radiale Scheiben gebildet wird. Der Katheter kann aus einem zylindrischen Rohrstück gebildet sein und zur Führung eines Endoskops durch seinen Innenraum dienen.

Die WO 97/14365 beschreibt einen Besamungskatheter mit einer Hülle, deren Ende von einem Fenster abgeschlossen ist. Innerhalb der Hülle ist ein optischer Lichtleiter angeordnet. Entlang der Hülle erstreckt sich eine Führungssonde, die eine Auslassöffnung aufweist. Am zweiten Anschluss eingespritzte Flüssigkeit wird die Hülle längs passieren und an der Austrittsöffnung austreten.

Die US 5,624,399 beschreibt einen Katheter, dessen distales Ende einen aufblasbaren Ballon trägt. In dem Katheter kann eine Leitung angeordnet sein.

Die EP 1177776 B1 beschreibt einen Besamungskatheter, der insbesondere zur Überwindung der ineinandergreifenden Gewebsfalten des Zervikalkanals von beispielsweise Rindern und kleinen Wiederkäuern geeignet sein soll, um tief im Uterus besamen zu können. Der Katheter weist ein Rohr auf, auf dessen Außenseite eine Spirale vorgesehen ist, und durch welches ein flexibler Schlauch vorgeschoben werden kann, der vorzugsweise flexible Längsrippen aufweist.

Es ist bekannt, dass bei der künstlichen Besamung die erforderlichen Spermienzahlen verringert werden können, wenn tief ins Uterushorn besamt wird, vorzugsweise beidseitig. Bisher konnte eine weitere Reduktion der Spermienzahlen, z.B. am Schwein und Schaf nur dadurch erreicht werden, dass Spermatozoen chirurgisch direkt in den Eileiter eingebracht wurden. Eine nicht-chirurgische Besamung mit geringeren Spermienzahlen als sie bei der Besamung tief ins Uterushorn erforderlich sind, ist bisher nicht bekannt.

### Aufgabe der Erfindung

Gegenüber dem Stand der Technik ist es Aufgabe der Erfindung, eine Kathetervorrichtung bereitzustellen, mit der nicht-chirurgisch Flüssigkeiten, insbesondere solche mit einem Gehalt an Gameten, bis in oder aus dem Eileiter des weiblichen Genitaltrakts transportiert werden können, sowie ein Verfahren zum nicht-chirurgischen Transport von Flüssigkeiten in einen oder aus einem Eileiter, sowie ein nicht-chirurgisches Verfahren zum Gametentransfer unter Verwendung der erfindungsgemäßen Kathetervorrichtung bereitzustellen. Die bereitzustellende Kathetervorrichtung soll insbesondere geeignet sein, um den Genitaltrakt eines weiblichen Wiederkäuers ohne Gewebsverletzung bis zum Eileiter zu passieren, um insbesondere beim Rind sowohl die ineinandergreifenden Faltungen der Zervix als auch den faltenreichen Uterus sowie ein daran anschließendes Uterushorn mit wendelförmigem bzw. widderhornartigem Verlauf zu überwinden und ein Ende in der utero-tubalen Verbindung bzw. im Eileiter zu positionieren.

### Allgemeine Beschreibung der Erfindung

Die Erfindung löst diese Aufgabe mit einer Kathetervorrichtung zum nicht-chirurgischen Gametentransfer gemäß den Ansprüchen. Insbesondere bezieht sich die Erfindung auf eine Vorrichtung, die für den nicht-chirurgischen Transport von Spermatozoen und/oder Oozyten in den Eileiter geeignet ist und die Verwendung der Kathetervorrichtung als Fertilisierungsvorrichtung beim Rind.

Ein Grund dafür, dass bisher kein nicht-chirurgisches Verfahren zur Einbringung von Spermatozoen in den Eileiter eines Säugetiers bekannt ist, wird darin gesehen, dass die Anatomie, insbesondere beim Rind, keine Einführung eines Besamungskatheters aus dem Uterushorn in den Eileiter erlaubt, weil Uterushorn und angrenzender Eileiter nicht geradlinig miteinander verbunden sind und überdies die utero-tubale Verbindung nur einen sehr kleinen Durchmesser aufweist.

Die erfindungsgemäße Kathetervorrichtung kann wahlweise zumindest ein Halteelement aufweisen, das an einem starren Führungselement angeordnet ist, in welchem eine flexible Leitung längsverschieblich angeordnet ist. Das Halteelement, auch als erstes Halteelement bezeichnet, wird mit einem terminalen Abschnitt der Zervix, in deren vaginalseitigen oder uterusseitigen Abschnitt, in Eingriff gebracht oder gegen diesen geordnet, sodass bei Belastung des Führungselements in eine vom Uterus weg weisende Richtung, die Zervix und/oder der Uterus durch die Belastung der Zervix gestreckt wird. Das erfindungsgemäße Verfahren verwendet daher optional den Eingriff des ersten Halteelements mit einem terminalen Abschnitt der Zervix, insbesondere einem vaginalseitigen terminalen Abschnitt der Zervix, um deren Falten zu strecken und die Durchführung des Führungselements oder eines längsverschieblich oder endständig am Führungselement angeordneten Ansatzstücks und/oder einer längsverschieblich im Führungselement angeordneten ersten flexiblen Leitung durch die Zervix zu erleichtern. Wahlweise wird beim Verfahren das erste Halteelement gegen den uterusseitigen terminalen Abschnitt der Zervix angeordnet, um den Uterus zu strecken. In der Folge sind einerseits die inneren Falten des Uterus gestreckt und daher geglättet, und andererseits wird der Zugang zum Eileiter durch das Uterushorn für eine flexible Leitung zugänglich, die im Führungselement verschieblich angeordnet ist. Entsprechend kann nach Streckung des Uterus die flexible Leitung (auch als Katheterschlauch bezeichnet) durch das Führungselement geschoben werden und durch den Uterus und durch das Uterushorn bis in den Eileiter vorgeschoben werden und Flüssigkeit kann in den Eileiter eingebracht werden, z.B. Spermatozoen und/oder Oozyten enthaltende Flüssigkeit, oder Flüssigkeit kann für die anschließende diagnostische Verwendung aus dem Eileiter entnommen werden. Wahlweise kann das Führungselement zusätzlich oder alternativ zu einem Halteelement ein Außengewinde bzw. eine Außenspirale aufweisen, die eine Einführung in die Zervix unter Drehung erleichtert, insbesondere zur Verwendung beim Schwein oder Schaf.

Optional tritt das optionale Halteelement mit einem vaginalseitigen terminalen Abschnitt der Zervix in Eingriff und dieser Abschnitt wird vom Uterus weg belastet, um die Zervix zu strecken, wobei während der Streckung der Zervix ein längsverschieblich oder endständig am Führungselement angeordnetes Ansatzstück durch die Zervix bewegt wird. Bei Positionierung des dem ersten, proximalen Ende gegenüberliegenden zweiten Endes des Ansatzstücks innerhalb des Uterus wird eine erste flexible Leitung durch das Führungselement, optional durch das Ansatzstück, in den Uterus bewegt. Erfindungsgemäß bevorzugt ist ein Mantel längsverschieblich im Führungselement angeordnet, wobei die erste und die koaxial darin angeordnete zweite flexible Leitung entlang des Mantels und längsverschieblich in diesem angeordnet sind.

Vorzugsweise weist ein solcher Mantel eine Sensorfläche einer Detektionseinrichtung auf, deren Datenleitung entlang des Mantels bis zu dessen erstem Ende geführt sein kann, welches auch als proximales Ende bezeichnet wird und z.B. im Bereich des ersten Endes des Führungselements bzw. im Bereich des proximalen Endes der flexiblen Leitung angeordnet ist. Dabei ist die Sensorfläche an dem dem ersten Ende des Mantels gegenüberliegenden zweiten oder distalen Ende des Mantels angeordnet und weist z.B. entlang der Längsachse des Mantels, so dass die Positionierung des zweiten Mantelendes optisch kontrollierbar ist. In dieser Ausführungsform kann der Mantel eine Steuereinrichtung, z.B. einen Steuerdraht aufweisen. Bevorzugt weisen erste und zweite flexible Leitung keine Steuereinrichtung, z.B. keinen Steuerdraht auf, so dass die erste und zweite flexible Leitung ausschließlich durch Verschiebung längs des Mantels beweglich sind und die zweite flexible Leitung ausschließlich durch Verschiebung längs innerhalb der ersten flexiblen Leitung beweglich ist, so dass die zweite flexible Leitung aus dem distalen Ende der ersten flexiblen Leitung heraus verschieblich ist.

Für die Zwecke der Erfindung liegen die ersten Enden der Elemente der Vorrichtung generell an oder in einem Abstand vor dem ersten Ende des Führungsrohrs, das beim Verfahren dem Uterus gegenüberliegt und daher auch als proximale Enden oder Abschnitte bezeichnet werden, während die im Verfahren jenseits der Zervix anzuordnenden Enden bzw. Abschnitte von Elementen als zweite bzw. distale bezeichnet werden. So ist z.B. ein Behälter, aus dem Flüssigkeit, insbesondere mit geschlechtschromosomenspezifisch sortierten Spermatozoen, in die zweite flexible Leitung eingebracht werden kann, oder in den Flüssigkeit aus dem Eileiter gesaugt werden kann, am proximalen bzw. ersten Ende der zweiten flexiblen Leitung angeschlossen, während die im Eileiter positionierbare Öffnung der zweiten flexiblen Leitung am gegenüberliegenden distalen bzw. zweiten Ende der zweiten Leitung angeordnet ist.

Ein Verfahren zum Transfer von Eizellen und/oder Spermazellen in den Eileiter oder die utero-tubale Verbindung kann unter Verwendung der bevorzugten Ausfiihrungsform der Kathetervorrichtung mit der erforderlichen Präzision der Positionierung des zweiten oder distalen Endes der zweiten flexiblen Leitung im Eileiter, in dessen Isthmus oder in der utero-tubalen Verbindung erfolgen, z.B. mit einer Abweichung von maximal 1 cm beim Rind, wobei Eizellen und/oder Spermazellen aus dem im Eileiter, in dessen Isthmus oder in der utero-tubalen Verbindung positionierten zweiten Ende der zweiten flexiblen Leitung austreten gelassen werden. Vorzugsweise wird eine Kathetervorrichtung als Fertilisierungsvorrichtung beim Rind verwendet, die die folgenden Elemente aufweist oder daraus besteht:
- ein starres Führungselement mit einem ersten Ende und einem am gegenüberliegenden zweiten Ende angeordneten oder durch das zweite Ende des Führungselements verschiebliches Ansatzstück, das sich in der Längsachse des Führungselements erstreckt, wobei am zweiten Ende des Führungselements ein optionales erstes Haltelement und/oder am zweiten Ende des Ansatzstücks ein optionales zweites Halteelement angeordnet ist, welches jeweils mit einem terminalen Abschnitt der Zervix in Eingriff bringbar ist oder gegen einen terminalen Abschnitt der Zervix angeordnet werden kann,
- eine axial im Führungselement angeordnete und axial im Führungselement verschiebliche erste flexible Leitung, deren proximales Ende jenseits des ersten Endes des Führungselements angeordnet ist und deren distales Ende durch das zweite Ende des Führungselements bzw. durch das Ansatzstück vorschiebbar ist,
- eine zumindest abschnittsweise entlang der ersten flexiblen Leitung angeordnete Detektionseinrichtung, deren Sensorfläche in einem distalen Abschnitt der ersten flexiblen Leitung angeordnet ist, der gegenüber deren proximalen Ende angeordnet ist,
- eine axial in der ersten flexiblen Leitung angeordnete zweite flexible Leitung, deren erstes Ende am oder jenseits des proximalen Endes der ersten Leitung zugänglich ist und die axial in der ersten flexiblen Leitung verschieblich ist, so dass ihr zweites Ende aus dem distalen Ende der ersten flexiblen Leitung heraus beweglich ist,
- optional eine zwischen erster flexibler Leitung und Führungselement und/oder Ansatzstück angeordnete Führungseinrichtung, z.B. als Rohr ausgebildet, die axial innerhalb des Führungselements festgelegt ist und in welcher die erste flexible Leitung axial verschieblich angeordnet ist,
- optional einen Mantel mit einem ersten Ende, das auf Seiten des ersten Endes des Führungselements angeordnet ist, in welchem Mantel die erste flexible Leitung längsverschieblich geführt ist, wobei der Mantel an seinem, dem ersten Ende gegenüberliegenden zweiten Ende eine Sensorfläche einer Detektionseinrichtung aufweist, wobei die Sensorfläche vorzugsweise an eine Datenleitung in dem Mantel, insbesondere an eine längs des Mantels verlaufenden Datenleitung angeschlossen ist.

Dabei umfasst das Verfahren
- die Anordnung des Führungselements der Kathetervorrichtung an die Zervix, optional mit dem Schritt des Eingriffs des ersten Halteelements mit einem terminalen vaginalseitigen Abschnitt der Zervix und Strecken der Zervix durch Belasten vom Uterus weg,
- das Positionieren des Ansatzstücks durch die Zervix und zumindest teilweise in den Uterus, z.B. durch Vorschieben des Ansatzstücks durch das Führungselement, optional mit dem Schritt des Eingriffs des zweiten Halteelements mit einem uterusseitigen terminalen Abschnitt der Zervix,
- optional das Vorschieben des Mantels, in welchem längsverschieblich die erste flexible Leitung und in dieser koaxial und/oder längsverschieblich die zweite flexible Leitung angeordnet ist, unter optischer Kontrolle mittels der am zweiten Ende des Mantels angeordneten Sensorfläche durch den Uterus, wobei vorzugsweise das Führungselement keine Belastung auf die Zervix vom Uterus weg ausübt, bis das zweite Ende des Mantels am oder im Uterushorn positioniert ist, insbesondere bis die Sensorfläche das Uterushorn detektiert, und/oder Vorschieben der ersten flexiblen Leitung, in der koaxial die zweite flexible Leitung angeordnet ist, unter optischer Kontrolle mittels der am distalen Abschnitt der ersten flexiblen Leitung angeordneten Sensorfläche durch den Uterus, wobei vorzugsweise das Führungselement keine Belastung auf die Zervix vom Uterus weg ausübt, bis das distale Ende der ersten flexiblen Leitung durch das Uterushorn bewegt ist,
- Vorschieben der ersten flexiblen Leitung, optional längs des Mantels über dessen zweites Ende hinaus, insbesondere in bzw. durch das Uterushorn bis vor die utero-tubale Verbindung, vorzugsweise unter optischer Kontrolle mittels einer Sensorfläche, die am distalen Abschnitt der ersten flexiblen Leitung angeordnet ist, wobei eine Sensorfläche am zweiten Ende des optionalen Mantels und/oder am distalen Ende der ersten flexiblen Leitung angeordnet sein kann,
- Vorschieben der zweiten flexiblen Leitung längs der ersten flexiblen Leitung aus deren distalem Ende hinaus bis zur Positionierung des zweiten Endes der zweiten flexiblen Leitung in der utero-tubalen Verbindung, vorzugsweise bis in den Eileiter, insbesondere in dessen Isthmus, insbesondere unter optischer Kontrolle einer Sensorfläche, die am distalen Abschnitt, insbesondere am distalen Ende der ersten flexiblen Leitung angeordnet ist,
- Transportieren einer Zusammensetzung, die Eizellen und/oder Spermien enthält, die insbesondere im Wesentlichen geschlechtsspezifisch sortiert sind, deren Population einheitlich nur ein X-Chromosom oder ein Y-Chromosom enthält, aus dem zweiten Ende der zweiten flexiblen Leitung oder Transportieren einer Zusammensetzung in das zweite Ende der zweiten flexiblen Leitung,
- Herausziehen der Vorrichtung, insbesondere durch Bewegen der ersten und zweiten flexiblen Leitung durch Ausüben von Zug auf das proximale Ende der ersten Leitung und/oder Ausüben von Zug auf das erste Ende der zweiten flexiblen Leitung, nachfolgend Ausüben von Zug auf das erste Ende des Mantels und Ausüben von Zug auf das erste Ende des Führungselements, mit Lösen des Eingriffs eines optionalen Halteelements von einem terminalen Abschnitt der Zervix.

Entsprechend betrifft die Erfindung die Verwendung der Kathetervorrichtung als Fertilisierungsvorrichtung zur Anordnung ausschließlich durch die natürlichen Öffnungen des Genitaltrakts eines weiblichen Säugetiers zur Positionierung des distalen Endes der ersten flexiblen Leitung in der utero-tubalen Verbindung und zur Positionierung des zweiten Endes der zweiten flexiblen Leitung in den Einleiter zum intratubalen Transfer von Eizellen und/oder Spermatozoen.

Bevorzugt weist die erste flexible Leitung einen Außendurchmesser von 1,4 bis 2,2 mm auf, bevorzugter von ca. 1,8 mm und einen Innendurchmesser von 1,0 bis 2,0 mm, bevorzugter von ca. 1,2 mm. Die erste flexible Leitung hat vorzugsweise eine Biegsamkeit bzw. Elastizität, die bei einer freien Länge von 50 mm eine maximale axiale Widerstandskraft bzw. Knicklast (nach Euler, 3. Knickfall) von 1500 bis 2500 mN, insbesondere von 1900 bis 2100 mN, z.B. von 2000 mN gegen das Knicken aufweist bzw. aufnimmt, und/oder bei einer freien Länge von 100 mm eine Widerstandskraft von 250 bis 800 mN, insbesondere von ca. 500 bis 600 mN, z.B. von 580 mN.

Die zweite flexible Leitung weist vorzugsweise an ihrem zweiten Ende einen Endabschnitt auf, der zu einer Spitze ausgezogen ist und der aus dem distalen Ende der ersten flexiblen Leitung herausbewegt werden kann. Dieser zu einer Spitze ausgezogene Endabschnitt hat vorzugsweise einen Außendurchmesser von 0,3 bis 0,7 mm, insbesondere von ca. 0,5 mm, und einen Innendurchmesser von ca. 0,1 bis 0,4 mm, bevorzugt von 0,2 mm, bei einer Länge von ca. 3 bis 8 cm, insbesondere von 4 bis 5 cm. Vorzugsweise hat dieser zu einer Spitze ausgezogene Endabschnitt eine Biegsamkeit bzw. Flexibilität mit einer maximalen axialen Widerstandskraft bzw. Knicklast (nach Euler, 3. Knickfall) bei einer freien Länge von 10 mm von 500 bis 1000 mN, insbesondere von 700 mN auf und/oder bei einer freien Länge von 17,5 mm von 80 bis 240 mN, insbesondere von 1600 mN, und/oder bei einer freien Länge von 25 mm von 70 bis 220 mN, insbesondere von 1500 mN, und/oder bei einer freien Länge von 35 mm von 20 bis 80 mN, insbesondere von 45 bis 50 mN. Anschließend an diesen Endabschnitt hat die zweite flexible Leitung vorzugsweise einen Außendurchmesser von 0,8 bis 1,8 mm, insbesondere von 1 mm, und einen Innendurchmesser von 0,2 bis 1,4 mm, insbesondere von 0,5 mm, und dieselbe Biegsamkeit oder Flexibilität, oder höhere Biegsamkeit, z.B. eine maximale Knicklast von 1000 mN.

Erfindungsgemäß bevorzugt weist die erste flexible Leitung an ihrem distalen Abschnitt, der an das distale Ende angrenzt oder gleich dem distalen Ende ist, eine Sensorfläche einer Detektionseinrichtung auf, die die optische Kontrolle der Positionierung des distalen Abschnitts bzw. des distalen Endes der ersten flexiblen Leitung erlaubt. Nach Positionierung des distalen Endes der ersten flexiblen Leitung unter optischer Kontrolle durch die Detektionseinrichtung angrenzend an die uterusseitige utero-tubale Verbindung wird erfindungsgemäß eine in der ersten flexiblen Leitung längsverschieblich angeordnete zweite flexible Leitung in Richtung auf die utero-tubale Verbindung bis in den Einleiter bewegt. Entsprechend wird die zweite flexible Leitung daher vom zweiten Ende des Führungselements weg bewegt, so dass das distale bzw. zweite Ende der zweiten flexiblen Leitung in den Eileiter bewegt wird.

Die erfindungsgemäße Vorrichtung ermöglicht erstmals insbesondere durch die Steifheit des Führungselements und des optionalen Ansatzstücks eine Einführung durch die Zervix und durch die hohe Biegsamkeit bzw. geringe maximale Knicklast von erster und zweiter flexibler Leitung ein Passieren des Uterus und des Uterushorns mit optischer Kontrolle mittels der Detektionseinrichtung durch Vorschieben dieser Leitungen, und durch das Vorschieben der zweiten flexiblen Leitung längs aus der ersten flexiblen Leitung das Positionieren des zweiten Endes der zweiten flexiblen Leitung in der utero-tubalen Verbindung, bevorzugt in den Eileiter.

Am ersten Ende der zweiten Leitung ist ein Behälter anschließbar, z.B. ein Spermatozoen enthaltender Behälter, die insbesondere geschlechtschromosomenspezifisch sortiert sind, mit einer Pumpe oder anderen Fördereinrichtung zum Transport des Behälterinhalts durch die zweite flexible Leitung bis an deren Öffnung an ihrem zweiten Ende, die z.B. die Querschnittsöffnung ist.

Das Führungselement kann einen geschlossenen Umfang aufweisen und z.B. rohrförmig sein oder eine längs verlaufende Öffnung aufweisen, z.B. spiralförmig oder achsparallel, und insbesondere einen U-förmigen Querschnitt aufweisen. Vorzugsweise ist das Führungselement aus Metall.

Das erste Halteelement ist mit einem terminalen Abschnitt der Zervix in Eingriff bringbar, insbesondere mit einem vaginalseitigen Abschnitt der Zervix. In diesen Ausführungsformen ist das Führungselement vorzugsweise so bemessen, dass es zumindest eine ausreichende Länge zur Anordnung des ersten Halteelements am vaginalseitigen terminalen Abschnitt der Zervix aufweist. Vorzugsweise ist das erste Halteelement eine Saugglocke, insbesondere mit ringförmigem Querschnitt, weist Saugöffnungen auf, oder weist auf oder besteht aus einem oder mehreren Zangengreifern.

In Ausführungsformen, in denen das erste Halteelement bei Anordnung des Führungselements durch die Zervix mit dem uterusseitigen terminalen Abschnitt der Zervix in Eingriff treten soll, kann das erste Halteelement alternativ ein aufblasbarer Ballon sein oder daraus bestehen, der ringförmig um das Führungselement angeordnet ist. In solchen Ausführungsformen ist das Führungselement vorzugsweise so bemessen, dass es zumindest eine ausreichende Länge zur Anordnung des ersten Halteelements am uterusseitigen terminalen Abschnitt der Zervix aufweist.

Für die Betätigung des ersten Halteelements ist eine erste Betätigungseinrichtung am, vorzugsweise im Führungselement angeordnet, die vom ersten Ende des Führungselements zugänglich und manipulierbar ist, während sie mit ihrem gegenüberliegenden zweiten Ende mit dem ersten Halteelement verbunden ist und dessen Zustand kontrolliert, z.B. in einen ersten Zustand, in welchem das erste Halteelement mit einem terminalen Abschnitt der Zervix in Eingriff steht, so dass die Zervix bei Belastung vom Uterus weg bewegt wird, um die Zervix zu strecken, wobei optional der Uterus gestreckt wird, und in einen zweiten Zustand, in welchem das erste Halteelement nicht mit der Zervix in Eingriff steht.

In bevorzugter Ausfiihrungsform weist die Kathetervorrichtung ein am zweiten Ende des Führungselements angeordnetes oder längsverschieblich und/oder drehbar im Führungselement angeordnetes Ansatzstück auf, durch welches die flexible Leitung geführt ist. Das Ansatzstück kann mit seinem ersten Ende am Führungselement angeordnet sein, beispielsweise am zweiten Ende des Führungselements befestigt sein, oder das Ansatzstück ragt bis in oder durch das Führungselement, sodass das erste Ende des Ansatzstücks innerhalb des Führungselements liegt, oder aus dem ersten Ende des Führungselements herausragt, wobei vorzugsweise das Ansatzstück drehbar und/oder längsverschieblich im Führungselement angeordnet ist. Das Ansatzstück dient insbesondere bei der Verwendung der Vorrichtung als Transferkathetervorrichtung oder als Kathetervorrichtung für den Gametentransfer dazu, die flexible Leitung während der Einbringung in die Zervix zu umgeben. Entsprechend findet das Führungselement Verwendung zur Anordnung durch den weiblichen Genitaltrakt zur Anordnung des ersten Halteelements bis zu einem endständigen (vaginalseitigen) Abschnitt der Zervix, oder zur Anordnung durch die Zervix zur Positionierung des ersten Halteelements am endständigen uterusseitigen Abschnitt der Zervix, während das Ansatzstück zur Anordnung durch die Zervix und/oder innerhalb des Uterus dient. Das Ansatzstück umgibt die flexible Leitung und bildet für die flexible Leitung eine Führung. Vorzugsweise ist das Ansatzstück starr.

Das Ansatzstück dient zur Führung bzw. Stabilisierung der flexiblen Leitung im Anschluss an das Führungselement. In bevorzugter Ausführungsform weist das Ansatzstück an seinem, dem Führungselement gegenüberliegenden zweiten Ende ein zweites Halteelement auf, das mit den uterusseitigen endständigen Abschnitten der Zervix in Eingriff bringbar ist. Das zweite Halteelement kann so ausgebildet sein wie das erste Halteelement, oder alternativ oder zusätzlich einen aufblasbaren ringförmigen Ballon aufweisen, der uterusseitig gegen den endständigen Abschnitt der Zervix anordnbar ist. So kann das zweite Halteelement einen oder mehrere, in einen Winkel von maximal 90° gegen die Längsachse des Ansatzstücks schwenkbare Arme, gegen das Ansatzstück abspreizbare Spangenelemente und/oder einen ringförmig aufblasbaren Ballon aufweisen oder daraus bestehen.

In den Ausführungsformen der Erfindung ist das optionale erste Halteelement mit einem der endständigenAbschnitte, insbesondere dem vaginalseitigen Abschnitt, der Zervix in Eingriff bringbar, optional ist das zweite Halteelement, das an dem zweiten Ende des Ansatzstücks gegenüber des Führungselements angeordnet ist, gegen den oder an dem uterusseitigen endständigen Abschnitt der Zervix anordnbar, sodass nach Eingriff des ersten Halteelements, optional zusätzlich nach Eingriff des zweiten Halteelements, zumindest ein endständiger Abschnitt der Zervix, optional beide endständigen Abschnitte der Zervix, mit der Vorrichtung in Eingriff stehen, sodass eine Belastung der Vorrichtung vom Uterus weg zu dessen Streckung führt und ein Ende der ersten und/oder der zweiten flexiblen Leitung, die durch das Führungselement geführt ist, durch den Uterus bis in den Eileiter angeordnet werden kann, wobei das zweite Ende der zweiten flexiblen Leitung bis in den Eileiter bewegt wird.

Wie das erste Halteelement, das am Führungselement angeordnet ist, ist auch das optionale zweite Halteelement des Ansatzstückes mit einer diesem zugeordneten Betätigungseinrichtung verbunden, die entlang des Führungselements und des Ansatzstücks, vorzugsweise innerhalb dieser angeordnet ist. Eine solche zweite Betätigungseinrichtung ist mit dem zweiten Halteelement verbunden und kann wie die erste Betätigungserrichtung ausgeführt sein, z.B. als Öffnungs- und Schließmechanik oder als flexible Leitung zur Zuführung bzw. zum Ablassen von Medium beim Aufpumpen bzw. Ablassen des Aufblasmediums, wenn das erste bzw. zweite Halteelement einen aufblasbaren ringförmigen Ballon aufweist.

Vorzugsweise ist ein Ansatzstück innerhalb des Führungselements angeordnet, sodass das erste Ende des Ansatzstücks im Bereich des ersten Endes des Führungselements angeordnet ist, wobei das erste Ende des Führungselements und das erste Ende des Ansatzstücks jeweils Griffstücke aufweisen, die getrennt voneinander bedienbar sind, sodass im Bereich des ersten Endes des Führungselements das Führungselement und das Ansatzstück separate Betätigungseinrichtungen aufweisen und gegeneinander drehbar und/oder gegeneinander längsverschieblich sind.

Vorzugsweise weist das Führungselement eine Führungseinrichtung auf, die z.B. achsparallel, vorzugsweise koaxial im Führungselement angeordnet ist, und in welcher die flexible Leitung längsverschieblich und drehbar anordnbar ist. Das Ansatzstück kann in der Führungseinrichtung angeordnet und geführt sein, insbesondere längsverschieblich in der Führungseinrichtung geführt sein. Die flexible Leitung ist vorzugsweise durch das Ansatzstück angeordnet und ragt über das erste Ende des Führungselements und über das erste Ende des Ansatzstücks hinaus und ist von Seiten des ersten Endes des Führungselements für die Drehung und/oder Längsverschiebung zugänglich. Wahlweise weist die flexible Leitung im Bereich ihres ersten Endes, das vorzugsweise im Bereich des ersten Endes des Führungselements angeordnet ist, eine Manipulationseinrichtung auf, beispielsweise einen Griff. Die Führungseinrichtung, die z.B. ein Rohr sein kann, ist vorzugsweise mittels Abstandshaltern im Führungselement festgelegt. Die Abstandshalter sind zwischen Führungseinrichtung und Führungselement angeordnet und können z.B. als Dichtung ausgeführt sein. Die erste flexible Leitung ist in dieser Ausführungsform durch eine innerhalb des Führungselements durch Abstandshalter festgelegte Führungsvorrichtung angeordnet. Das erste Halteelement kann als terminaler Abschnitt von Führungselement und Führungseinrichtung ausgebildet sein, der von einem als Dichtung ausgeführten Abstandshalter abgegrenzt wird, insbesondere, wenn eine Leitung, die entlang des Führungselements angeordnet ist, als erste Betätigungseinrichtung mit diesem terminalen Abschnitt verbunden ist.

Bevorzugt ist innerhalb des Führungselements, z.B. innerhalb einer im Führungselement angeordneten Führungseinrichtung und/oder innerhalb eines am Führungselement, vorzugsweise in der Führungseinrichtung angeordneten Ansatzstücks ein Mandrinträger reversibel anordnbar, dessen zweites Ende einen die Querschnittsöffnung des Führungselements oder die Querschnittsöffnung des an diesem angeordneten Ansatzstücks oder die Querschnittsöffnung einer Führungseinrichtung überdeckenden Mandrin aufweist. Der Mandrinträger weist ein von Seiten des ersten Endes des Führungselements zugängliches erstes Griffende auf. Im erfindungsgemäßen Verfahren überdeckt der Mandrin während der Einführung bis an oder durch die Zervix die Querschnittsöffnung des Führungselements oder die Querschnittsöffnung des an diesem angeordneten Ansatzstücks oder die Querschnittsöffnung einer im Führungselement angeordneten Führungseinrichtung, um eine Verletzung des Säugetiers durch den Rand der Querschnittsöffnung zu vermeiden. Vorzugsweise wird der Mandrin nach Anordnen des zweiten Endes des Führungselements oder des an diesem angeordneten Ansatzstücks an einem terminalen Abschnitt der Zervix durch Bewegung des Mandrinträgers durch die Öffnung am ersten Ende des Führungselements aus dem Führungselement entfernt. Bevorzugt wird die flexible Leitung nach Entfernen des Mandrinträgers aus dem Führungselement in diesem, ggf. in der Führungseinrichtung und/oder im Ansatzstück angeordnet und nach Eingriff des ersten und/oder zweiten Halteelelements mit zumindest einem terminalen Abschnitt der Zervix und während einer Belastung der Zervix in eine vom Uterus weg gerichtete Richtung durch den Uterus vorgeschoben.

Die erste flexible Leitung ist biegsam, um z.B. außerhalb des Führungselements, außerhalb der Führungsvorrichtung oder außerhalb des Ansatzstücks eine gebogene Konformation einzunehmen, und ist vorzugsweise ausreichend starr, um gezielt vorschiebbar zu sein. Im erfindungsgemäßen Verfahren beim Gametentransfer bei Rindern ist die flexible Leitung aus dem Führungselement bis in den Uterus vorschiebbar, z.B. unter rektaler manueller Kontrolle, und kann daher generell auch als starre oder semi-flexible Leitung bezeichnet werden.

In bevorzugter Ausführungsform ist innerhalb der ersten flexiblen Leitung ein Schiebeelement verschieblich angeordnet, dessen proximales Ende in einem Bereich vor dem ersten Ende des Führungselements zugänglich ist, und dessen distales, gegenüberliegendes Ende einen weiteren oder zweiten Mandrin und/oder einen aufblasbaren Ballon tragen kann, der über das zweite Ende der ersten flexiblen Leitung hinausragt. Ein solcher Mandrin und/oder aufblasbarer Ballon ist zur Anordnung im Bereich der utero-tubalen Verbindung geeignet, um die Einführung der flexiblen Leitung in den Eileiter zu erleichtern.

Ein aufblasbarer Ballon am distalen terminalen Abschnitt der ersten flexiblen Leitung, der in den Eileiter einführbar ist, ist bevorzugt, um den Querschnitt des Eileiters vor, während und/oder nach Einbringen von Flüssigkeit temporär gegen den Uterus zu dichten. Für das Aufblasen dieses Ballons, der vorzugsweise ringförmig um das zweite Ende der flexiblen Leitung angeordnet ist, ist der Ballon mit einer längs der flexiblen Leitung angeordneten Leitung verbunden, welche mit Druck beaufschlagbar ist.

Weiterhin kann das Führungselement und/oder das Ansatzstück eine Abdichtungseinrichtung aufweisen, die das Führungselement gegen einen Abschnitt der Zervix abdichtet, sowie eine mit einer Druckquelle verbindbare Leitung, deren Öffnung uterusseitig von der Abdichtungseinrichtung, insbesondere in einem Abschnitt am zweiten Ende des Führungselements und/oder des Ansatzstücks endet, und daher bei der Verwendung innerhalb Uterus platziert wird. Die Abdichtungseinrichtung ist vorzugsweise expandierbar, beispielsweise durch Einführung eines Mediums aufblasbar, sodass sie zur Abdichtung der Zervix gegenüber des Führungselements bzw. des Ansatzstückes dient, und beim Verfahren durch die Leitung in den Uterus suffliertes Gas, insbesondere CO₂, nicht entweichen kann. Bei dieser Ausführungsform wird die Leitung, z.B. ein Schlauch, für die Insufflierung von Gas in den Uterus verwendet, und die Abdichtungsvorrichtung zur Abdichtung der Zervix gegen das Führungselement und/oder gegen das Ansatzstück. In Ausführungsformen, in denen das Führungselement und/oder das Ansatzstück ein an dessen zweitem Ende angeordnetes ringförmiges aufblasbares erstes bzw. zweites Halteelement aufweist, kann die Abdichtungseinrichtung entfallen und durch das erste bzw. zweite Halteelement ersetzt werden, das gegen den uterusseitigen terminalen Abschnitt der Zervix anordnbar ist. In dieser Ausführungsform weist die erfindungsgemäße Kathetervorrichtung zur Verwendung für das Insufflieren von Gas in den Uterus z.B. nur eine Leitung zur Zuführung von Gas auf, deren Öffnung auf der Seite der Halteeinrichtung angeordnet ist, die dem ersten Ende des Führungselements gegenüber liegt, und damit bei der Verwendung uterusseitig angeordnet wird.

Insbesondere bevorzugt ist die Verwendung der Kathetervorrichtung, bzw. ein Verfahren für die Einbringung von Spermatozoen und/oder Oozyten, insbesondere geschlechtsspezifisch sortierter Spermatozoen in einen Eileiterbereich hinter der utero-tubalen Verbindung mittels der Kathetervorrichtung, insbesondere in den Isthmus des Eileiters, insbesondere des Rindes.

Weiter bevorzugt weist die Vorrichtung eine längs der flexiblen Leitung angeordnete Steuereinrichtung, auch Führungsdraht genannt auf, die vorzugsweise im Bereich des ersten Endes des Führungselements zur Handhabung und Steuerung zugänglich ist. Die Steuereinrichtung ist mit der flexiblen Leitung innerhalb des Führungselements anordnbar.

Weiter bevorzugt weist die Vorrichtung eine entlang der flexiblen Leitung angeordnete Detektionseinrichtung auf, deren Sensorfläche in einem distalen Abschnitt der flexiblen Leitung angeordnet ist, der dem proximalen Ende der flexiblen Leitung am ersten Ende des Führungselements gegenüber liegt. Die Detektionseinrichtung ist vorzugsweise parallel zur flexiblen Leitung angeordnet und mit dieser durch das Führungselement anordnbar. Die Detektionseinrichtung kann z.B. eine Glasfaser aufweisen, deren Querschnitt die Sensorfläche ist. Alternativ kann die Sensorfläche ein Ultraschallkopf sein, der mit einer Datenleitung verbunden ist, die längs der flexiblen Leitung angeordnet ist.

Die flexible Leitung, auch als erste flexible Leitung bezeichnet, enthält erfindungsgemäß bevorzugt eine zweite flexible Leitung längsverschieblich, die vorzugsweise eine geringere Steifigkeit aufweist, als die erste flexible Leitung. Dabei ist bevorzugt, dass die eine der beiden flexiblen Leitungen eine über die Länge konstante Biegefestigkeit aufweist, während die andere flexible Leitung aneinander angrenzende Abschnitte, beispielsweise von je 2 bis 20 cm Länge aufweist, die jeweils eine unterschiedliche Biegefestigkeit haben. Vorzugsweise hat die zweite flexible Leitung abschnittsweise unterschiedliche Biegefestigkeiten und die äußere flexible Leitung, die insbesondere die erste flexible Leitung ist, weist eine konstante Biegefestigkeit auf. Insbesondere bevorzugt ist der distale bzw. zweite Endabschnitt der zweiten flexiblen Leitung aus der ersten flexiblen Leitung vorschiebbar und ist bogenförmig vorgekrümmt, wobei der den zweiten Endabschnitt der zweiten flexiblen Leitung umgebende Endabschnitt der ersten flexiblen Leitung eine höhere Steifigkeit aufweist. In dieser Ausführungsform hält die die zweite flexible Leitung enthaltende erste flexible Leitung den umfassten distalen Endabschnitt der zweiten flexiblen Leitung in einer im wesentlichen geraden Konformation, während in vorgeschobener Anordnung des distalen Endabschnitts der zweiten flexiblen Leitung aus der ersten flexiblen Leitung der distale Endabschnitt der zweiten flexiblen Leitung eine seiner Vorkrümmung entsprechende Konformation aufweist. Generell bevorzugt weist die zweite flexible Leitung einen geringen Außendurchmesser auf, so dass sie in die utero-tubale Verbindung einführbar ist, und ist elastisch verformbar, insbesondere mit einer geringen Steifigkeit bzw. einer Elastizität mit einer geringen maximalen Knicklast, z.B. von maximal 1000 mN, um Verletzungen der utero-tubalen Verbindung z.B. durch ein Einstechen zu vermeiden. Bevorzugt weist die zweite flexible Leitung zumindest an ihrem distalen Ende, das über das distale Ende der ersten flexiblen Leitung vorschiebbar ist, eine hohe elastische Verformbarkeit bei axialer Belastung auf, z.B. eine geringere maximale Knicklast als die erste flexible Leitung.

Zur Verringerung des inneren Volumens der flexiblen Leitung ist bevorzugt, dass die zweite flexible Leitung einen wesentlich geringeren inneren Querschnitt aufweist als die äußere flexible Leitung, beispielsweise Bereich von 0,2 bis 3 mm Innendurchmesser. Weiterhin weist vorzugsweise die flexible Leitung, insbesondere die darin angeordnete zweite flexible Leitung ein zu einer Spitze ausgezogenes distales (zweites) Ende auf. In Ausführungsformen, in denen eine zweite flexible Leitung in der flexiblen Leitung angeordnet ist, ist die Detektionseinrichtung vorzugsweise an der äußeren flexiblen Leitung angeordnet, sodass bei der Verwendung der Vorschub der zweiten flexiblen Leitung aus der äußeren flexiblen Leitung durch die Sensorfläche beobachtbar ist, die an der ersten flexiblen Leitung befestigt ist, die die längsverschiebliche zweite flexible Leitung umgibt.

Optional ist die erste und/oder die zweite flexible Leitung mit einem Führungsdraht versehen, der entlang dieser Leitung verläuft und von außerhalb des Führungsabschnitts gesteuert bewegbar ist, beispielsweise mit einer Manipulationseinrichtung, zum Beispiel einem Griff jenseits des ersten Endes des Führungselements versehen ist.

Generell bevorzugt sind die flexible Leitung, Detektionseinrichtung und ein Führungsdraht in Kanälen angeordnet, die in einem gemeinsamen Mantel verlaufen. Der gemeinsame Mantel ist elastisch und kann beispielsweise ein nach Vorschub und Krümmung steuerbarer Mantel sein.

Das erfindungsgemäße Verfahren sieht insbesondere vor, das distale Ende einer flexiblen Leitung bis in oder über die utero-tubale Verbindung hinweg vorzuschieben, bevorzugter bis in den Isthmus des Eileiters, weniger bevorzugt bis in die Ampulle des Eileiters, und Flüssigkeit austreten zu lassen oder zu entnehmen, insbesondere Spermatozoen und/oder Oozyten enthaltende Flüssigkeit einzubringen. Es hat sich gezeigt, dass bei Einbringen von Spermatozoen und/oder Oozyten, insbesondere von Spermatozoen, die geschlechtsspezifisch sortiert waren, bis in die utero-tubale Verbindung oder in den Isthmus des Eileiters auch mit Spermienzahlen zu Trächtigkeiten führt, die gegenüber Spermienzahlen, die bei der tief intrauterinen Besamung regelmäßig erforderlich sind, deutlich reduziert waren. Z.B. wurden bei Rindern für durchflusszytometrisch geschlechtsspezifisch sortierte Spermien mit Zahlen im Bereich von ca. 500 bis 500.000 vitale Spermien pro Eileiter, bevorzugter 1000 bis 100.000 für eine erfolgreiche Befruchtung von Färsen als ausreichend gefunden, während in Vergleichsversuchen mit gleichermaßen synchronisierten Färsen mit Aliquots des sortierten Spermas mindestens 1 Million Spermien erforderlich waren, wenn tief ins Uterushorn besamt wurde.

Entsprechend umfasst das erfindungsgemäße Verfahren vorzugsweise die geschlechtsspezifische Sortierung der von einem männlichen nicht-menschlichen Säugetier der selben Spezies gewonnenen Spermatozoen, insbesondere die durchflußzytometrische Sortierung nach dem Gesamt-DNA-Gehalt und/oder nach geschlechtschromosomenspezifischer Detektion einzelner Spermien, Herstellen einer Probe aus geschlechtschromosomenspezifischen Spermatozoen und Einbringen dieser mittels der erfindungsgemäßen Kathetervorrichtung in die utero-tubale Verbindung und/oder in den Eileiter des weiblichen Tiers, z.B. in den Isthmus des Eileiters. Weiter bevorzugt werden erfindungsgemäß Spermatozoen in einem geringen Volumen von beispielsweise 10 - 100 µL, insbesondere ca. 30 bis 50 µL Gesamtvolumen eingebracht, was negative Wirkungen der Verdünnung z.B. auf die Befruchtungsrate reduziert.

### Genaue Beschreibung der Erfindung

Die Erfindung wird nun genauer mit Bezug auf die Figuren beschrieben, die schematisch in
- Figur 1 eine Schnittansicht einer erfindungsgemäßen Vorrichtung,
- Figur 2 eine weitere Ausführungsform der Vorrichtung,
- Figur 3 ein Detail einer erfindungsgemäßen Vorrichtung,
- Figur 4 eine Übersicht über eine erfindungsgemäße Vorrichtung,
- Figur 5 eine erfindungsgemäße Vorrichtung bei der Verwendung im erfindungsgemäßen Verfahren,
- Figur 6 eine weitere Ausführungsform der Verwendung der erfindungsgemäßen Vorrichtung,
- Figur 7 eine weitere Ausführungsform der Verwendung bzw. des Verfahrens und
- Figur 8 eine weitere Ausführungsform zeigen.

In den Figuren bezeichnen gleiche Bezugsziffern funktionsgleiche Elemente.

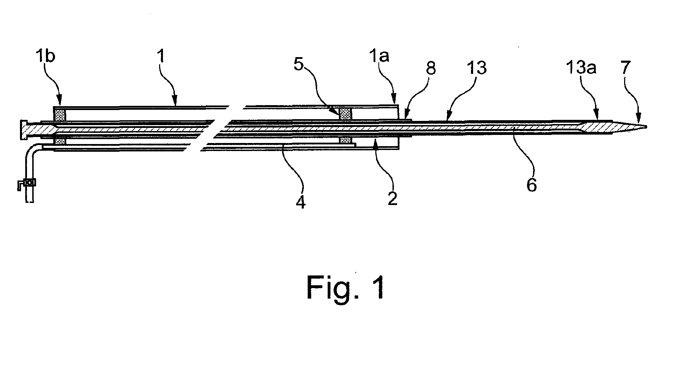

Figur 1 zeigt eine erfindungsgemäße Kathetervorrichtung mit einem Führungselement 1, das an seinem zweiten Ende 1a ein erstes Halteelement 2 aufweist, vorliegend als einfache Saugglocke gezeigt, die durch einen Abschnitt des Führungselements 1 gebildet wird, der von einer zwischen Führungseinrichtung 8 und Führungselement 1 angeordneten Dichtung 5 gebildet wird. Das Ansatzstück 13 ist in der Führungseinrichtung 8 längsverschieblich angeordnet. Beim Verfahren zum Gametentransfer wird nach Einführung des Führungselements in den Genitaltrakt des weiblichen Tiers bis in eine Position, in der das erste Halteelement 2 an einem terminalen Abschnitt der Zervix anliegt, vorzugsweise das Ansatzstück 13 bis in den Uterus geführt, insbesondere durch die Zervix. Während der Einführung des Führungselements 1 ist dessen Querschnittsöffnung am zweiten Ende 1a vorzugsweise von einem Mandrin (nicht gezeigt) überdeckt, der mit einem Mandrinträger verbunden ist, der im Führungselement 1, vorzugsweise in der Führungseinrichtung 8 angeordnet ist und durch die Querschnittsöffnung am ersten Ende 1b des Führungselements 1 entfernbar ist.

Vorzugsweise ist zumindest während des Schritts der Anordnung des Ansatzstücks 13 durch die Zervix die Querschnittsöffnung des zweiten Endes 13a des Ansatzstücks 13 von einem Mandrin 7 überdeckt. Die flexible Leitung 3 (nicht gezeigt) ist in die Führungseinrichtung, in der gezeigten Ausführungsform in das Ansatzstück 13 anordnbar, nachdem der Mandrin 7 und das Schiebeelement 6 aus dem Ansatzstück 13 entfernt sind.

Das erste Halteelement 2 ist mit einer ersten Betätigungseinrichtung 4 verbunden, deren eines Ende von Seiten des ersten Endes 1b des Führungselements 1 zugänglich ist. Vorliegend ist die erste Betätigungseinrichtung 4 als eine Leitung gezeigt, die das erste Halteelement 2 aus einem Bereich am ersten Ende 1b des Führungselements 1 zugänglich macht, beispielsweise in Form der Öffnung der Leitung, die die erste Betätigungseinrichtung 4 bildet, im Bereich des ersten Endes 1b des Führungselements 1.

Alternativ zu der gezeigten Saugeinrichtung kann das erste Halteelement 2 einen Ballonabschnitt bilden, der durch die erste Betätigungseinrichtung 4 aufblasbar ist. Beispielsweise kann in einer einfachen Ausführungsform das zweite Ende 1a des Führungselements 1 bis auf die flexible Leitung 3 und/oder bis auf das Ansatzstück 13 geschlossen sein, um, z.B. mit der Dichtung 5 zwischen Führungselement 1 und Führungseinrichtung 8, einen aufblasbaren ringförmigen Ballon (nicht gezeigt) am zweiten Ende 1a des Führungselements 1 zu bilden, das einen bis auf die flexible Leitung 3 und/oder bis auf das Ansatzstück 13 geschlossenen Endabschnitt aufweist.

Entsprechend der bevorzugten Ausführungsform ist im Ansatzstück 13 ein Schiebeelement 6 gezeigt, durch welches ein Mandrin 7 verschieblich ist, so dass z.B. nach Einführen des Ansatzstücks 13 durch die Zervix der Mandrin 7 aus dem Ansatzstück 13 entfernt werden und die flexible Leitung 3 in und durch das Ansatzstück 13 geführt werden kann. Alternativ oder zusätzlich zum gezeigten Mandrin 7 kann an dem distalen Ende 13a des Ansatzstücks 13 ein aufblasbarer Ballon angeordnet sein, beispielsweise ringförmig um einen Außenabschnitt am zweiten Ende 13a des Ansatzstücks 13, und mit einer Leitung längs des Ansatzstücks 13 zur Zu- und Abfuhr von Medium verbunden sein.
Vorzugsweise weist das Führungselement 1 eine Führungseinrichtung 8 auf, die vorzugsweise koaxial im Führungselement 1 angeordnet ist, und in welcher das Ansatzstück 13, in dem die flexible Leitung 3 längsverschieblich anordnbar ist, längsverschieblich und drehbar angeordnet ist. Das Ansatzstück 13 ragt vorzugsweise über das erste Ende 1b des Führungselements 1 hinaus, und ist von Seiten des ersten Endes 1b des Führungselements 1 für die Drehung und/oder Längsverschiebung zugänglich, und weist wahlweise eine Manipulationseinrichtung (nicht gezeigt) auf, beispielsweise einen Griff.

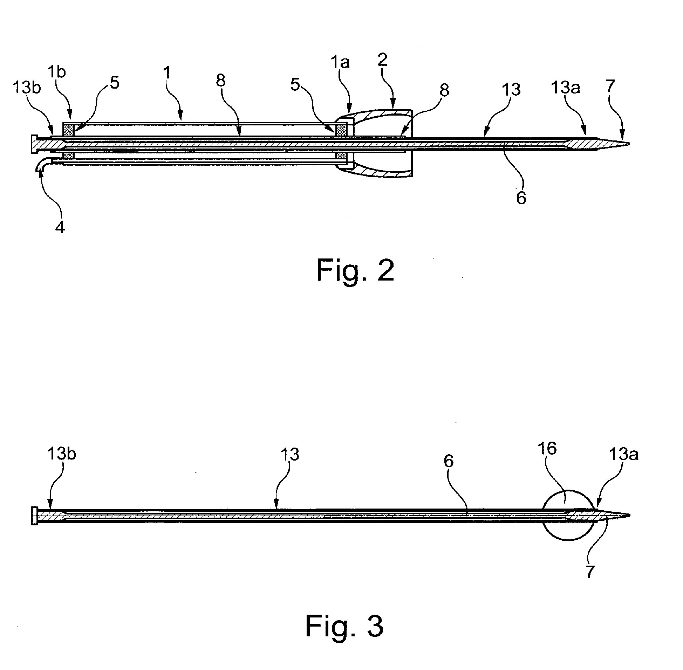

Figur 2 zeigt in Schnittansicht das Führungselement 1 der Kathetervorrichtung, an dessen zweitem Ende 1a ein erstes Halteelement 2 angeordnet ist, die mit einer ersten Betätigungseinrichtung 4 manipulierbar ist. Entsprechend einer bevorzugten Ausführungsform ist das erste Halteelement 2 eine Saugglocke, durch die mittels der ersten Betätigungseinrichtung 4, die beispielsweise eine längs des Führungselements 1 verlaufende Leitung ist, ein Unterdruck angelegt werden kann. Bei der Verwendung kann dieses erste Halteelement 2 an einen terminalen Abschnitt der Zervix Z angeordnet werden, und dieser Abschnitt wird bei Anlegen von Unterdruck an der ersten Betätigungseinrichtung 4 durch Unterdruck am ersten Halteelement 2 festgelegt, sodass durch Ausübung von Zug am Führungselement 1 eine Zugbelastung auf die Zervix Z ausgeübt werden kann, die vom Uterus U weg gerichtet ist.

Das Führungselement 1 weist eine zentrale und/oder koaxial angeordnete Führungseinrichtung 8 auf, die mit Dichtungen 5 gegen das Führungselement 1 abgedichtet ist. In der Führungseinrichtung ist längsverschieblich ein Ansatzstück 13 angeordnet, durch welches die flexible Leitung 3 (nicht gezeigt) nach Entfernung des mit dem Schiebeelement 6 gekoppelten Mandrins 7 anordnbar ist. Entsprechend ist das erste Halteelement 2 in der hier gezeigten Ausführungsform als eine am zweiten Ende 1a des Führungselements 1 angeordnete Saugglocke durch die am zweiten Ende 1a des Führungselements 1 angeordnete Dichtung 5 ausgebildet, die zwischen Führungselement 1 und Führungseinrichtung 8 angeordnet ist und einen Abschnitt des Führungselements 1 abgrenzt. Das Halteelement 2 ist durch die als Leitung ausgebildete erste Betätigungseinrichtung 4 vom ersten Ende des Führungselements 1 zugänglich, beispielsweise durch Anlegen von Unterdruck im erfindungsgemäßen Verfahren für den Eingriff mit einem terminalen Abschnitts der Zervix Z. Die flexible Leitung 3 ist entsprechend der generell bevorzugten Ausführungsform durch die im Führungselement 1 angeordnete Führungseinrichtung, insbesondere durch das in der Führungseinrichtung 8 längsverschieblich angeordnete Ansatzstück 13 längsverschieblich anordnbar. Für den Schritt der Einführung des Ansatzstücks 13 ist vorzugsweise ein Mandrin 7 am zweiten Ende 13a des Ansatzstücks 13 angeordnet, der mittels seines Schiebeelements 6 durch das erste Ende 1b des Führungselements 1 bzw. durch das an diesem angeordnete erste Ende 13b des Ansatzstücks 13 entfernbar ist.

Generell ist bevorzugt, dass sich die Führungseinrichtung 8 bis in den Bereich des ersten Halteelements 2 bzw. bis in den Bereich eines zweiten Halteelements 9 des Ansatzstücks 13 erstreckt.

Die flexible Leitung 3 ist z.B. so starr, dass sie ohne zusätzliche Versteifung aus dem Führungselement 1 bzw. aus dem Ansatzstück 13 vorschiebbar ist und hinreichend flexibel bzw. semi-flexibel, dass z.B. bei Kontaktierung gegen eine Gewebsoberfläche eine Biegung um maximal 90°, bevorzugt um 10 bis 45**°** auftritt und die flexible Leitung 3 bei weiterem Vorschub ausweicht.

Eine Saugglocke, die ein erstes Halteelement 2 bildet, wird vorzugsweise von einem trichter- oder glockenförmig am zweiten Ende 1a des Führungselements 1 angeordneten Material gebildet, das starr oder flexibel, z.B. gummielastisch sein kann. Für die einfache Sterilisierbarkeit der Vorrichtung, z.B. durch Autoklavieren oder Abflammen, z.B. nach Benetzen mit Alkohol, besteht das erste und ggf. ein zweites Halteelement 2, 9 sowie das Führungselement 1 und die optionale Führungseinrichtung 8 einschließlich Abstandshaltern 5 vorzugsweise aus temperaturbeständigem Material, insbesondere aus Edelstahl.

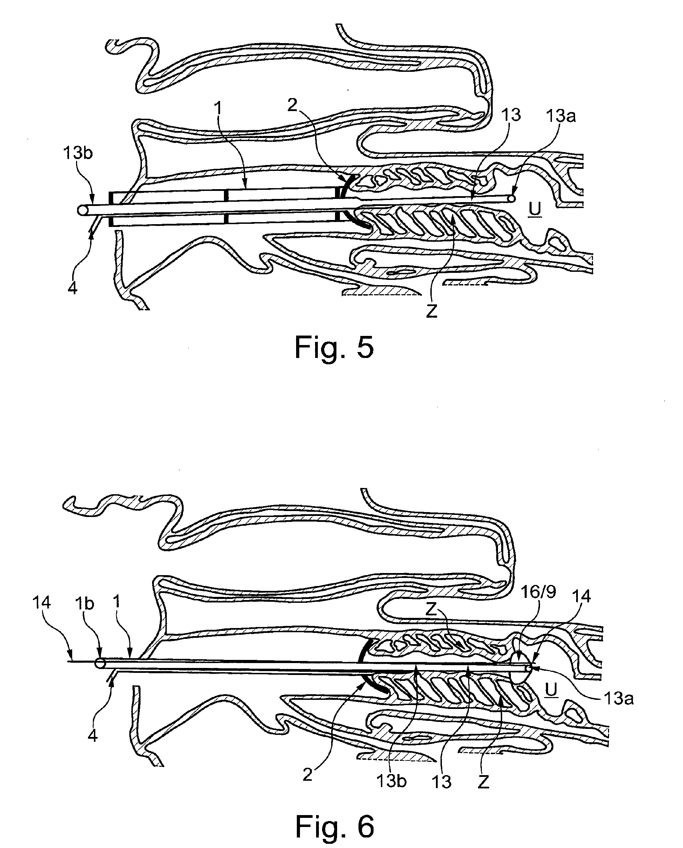

Generell ist bevorzugt, dass die flexible Leitung 3 eine Länge aufweist, die ausreicht, um im Verfahren ihr distales Ende 3a zumindest durch die Zervix bis in den Uterus, vorzugsweise bis an oder in die utero-tubale Verbindung vorzuschieben, während das Führungselement 1 bis an oder durch die Zervix Z geführt ist, wenn ein erstes Halteelement 2 und/oder ein zweites Halteelement 9 an einem terminalen Abschnitt der Zervix Z angeordnet ist, insbesondere, wenn ein vom Uterus weggerichteter Zug an der Zervix anliegt. Am Führungselement 1 kann ein Ansatzstück (nicht gezeigt, siehe Figuren 5 bis 8) angeordnet sein, welches vorzugsweise so bemessen ist, dass es die Zervix durchqueren kann. Die flexible Leitung 3 tritt bei ihrer Anordnung durch das Führungselement 1 am zweiten Ende des Ansatzstücks aus.

Figur 3 zeigt nur ein Ansatzstück 13 im Detail, in welchem ein Schiebeelement 6 mit endständigem Mandrin 7 angeordnet ist, der über das zweite Ende 13a des Ansatzstücks 13 hinausragt. Weiterhin weist entsprechend der bevorzugten Ausführungsform das Ansatzstück 13 optional an seinem zweiten Ende 13a einen am Außenumfang angeordneten ringförmigen aufblasbaren Ballon 16 auf, der mittels einer Leitung, die längs des Ansatzstücks 13 verläuft, für das Aufblasen und das Ablassen von Medium verbunden ist.

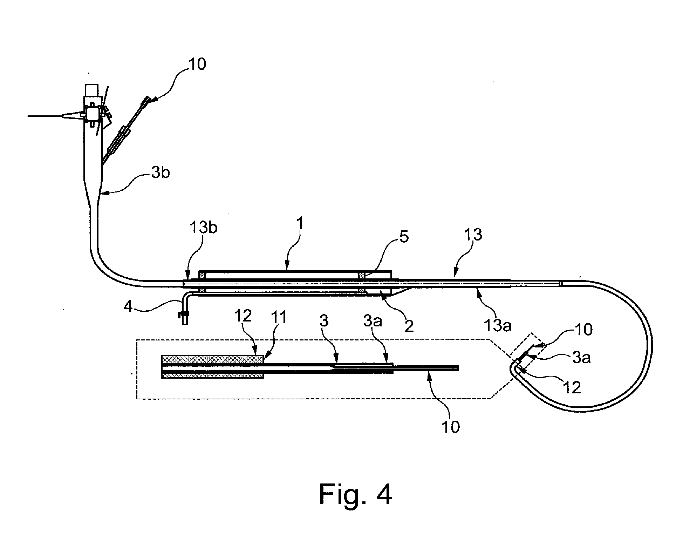

Figur 4 zeigt die Kathetervorrichtung in bevorzugter Ausführungsform, bei der eine Detektionseinrichtung, die beispielsweise der eines herkömmlichen Endoskops entspricht, entlang der flexiblen Leitung 3 angeordnet ist, und überdies innerhalb der flexiblen Leitung 3 eine zweite flexible Leitung 10 angeordnet ist, welche innerhalb der flexiblen Leitung 3 längsverschieblich ist. Vorzugsweise ist die flexible Leitung 3 innerhalb eines Mantels 12 längsverschieblich geführt. Die Sensorfläche 11 ist an einer Datenleitung (nicht gezeigt) der Detektionseinrichtung in einem Mantel 12 angeschlossen, in welchem auch die flexible Leitung 3 mit darin längsverschieblich angeordneter zweiter flexibler Leitung 10 angeordnet ist. Die flexible Leitung 3 kann im Mantel 12 längsverschieblich sein und überdies in einem Abstand vor der Sensorfläche 11 enden, oder bündig mit der Sensorfläche 11 abschließen, während die zweite flexible Leitung 10 vorzugsweise aus der flexiblen Leitung 3 vorschiebbar ist, und die Auslassöffnung der zweiten flexiblen Leitung 10 bis in einen Abstand vor die Sensorfläche 11 schiebbar ist, beispielsweise zur Anordnung der Auslassöffnung der zweiten flexiblen Leitung 10 innerhalb des Isthmus I des Eileiters E.

Figur 5 zeigt am Beispiel des schematisch dargestellten Genitaltrakts eines Rindes das Führungselement 1, das ein erstes Halteelement 2 in Form einer mit einer ersten Betätigungseinrichtung 4 verbundenen Saugglocke aufweist. Am zweiten Ende des Führungselements 1 ist ein Ansatzstück 13 angeordnet, dessen zweites Ende 13a in einem Abstand vor dem zweiten Ende 1a des Führungselements 1 angeordnet ist, während ein Abschnitt des Ansatzstücks 13 innerhalb des Führungselements 1 angeordnet ist, sodass das erste Ende 13b des Ansatzstücks 13 vom ersten Ende 1b des Führungselements 1 zugänglich ist und beispielsweise an seinem ersten Ende 13b mit einer Manipulationseinrichtung, beispielsweise einem Griff versehen ist. Das Ansatzstück 13 ist zur Anordnung durch die Zervix Z verwendbar, wenn die erste Betätigungseinrichtung 4 des Führungselements 1 an einem terminalen Abschnitt der Zervix Z angeordnet ist, z.B. wie hier dargestellt am vaginalseitigen terminalen Abschnitt. Die Fixierung eines terminalen Abschnitts der Zervix Z erfolgt vorliegend durch den Eingriff des ersten Halteelements 2 an einem terminalen Abschnitt der Zervix Z, hier durch Anlegen von Unterdruck an die erste Halteeinrichtung 2 durch die erste Betätigungseinrichtung 4, die eine Leitung zum Anlegen von Unterdruck und daher mit einer Unterdruckquelle verbunden ist.

Bei der Verwendung wird das zweite Ende 13a des Ansatzstücks 13 im Bereich des Uterus U nach Passage durch die Zervix Z angeordnet. Die weiteren Elemente der erfindungsgemäßen Vorrichtung sind in den Figuren 5 bis 8 nicht dargestellt, insbesondere nicht die flexible Leitung 3, eine Führungseinrichtung 8 und eine Detektionseinrichtung.

Figur 6 zeigt eine Ausführungsform, bei der am zweiten Ende 1a des Führungselements 1 ein Ansatzstück 13 befestigt ist, dessen Achse im Wesentlichen in der Achse des Führungselements 1 liegt. Das erste Halteelement 2 ist am zweiten Ende 1a des Führungselements 1 angeordnet und steht durch Betätigung der ersten Betätigungseinrichtung 4 mit dem vaginalseitigen terminalen Abschnitt der Zervix Z in Eingriff Das Ansatzstück 13 ist durch die Zervix Z angeordnet und weist an seinem zweiten Ende 13a ein zweites Halteelement 9 auf, das mit einer zweiten Betätigungseinrichtung 15 verbunden ist, die längs des Ansatzstücks 13 und des Führungselements 1 angeordnet ist. Das zweite Halteelement 9 kann gegen die Längsachse des Ansatzstücks 13 abspreizbare Spangen und/oder einen aufblasbaren ringförmigen Ballon 16 aufweisen. Durch Betätigung des zweiten Halteelements 9 mittels der zweiten Betätigungseinrichtung 15 tritt das zweite Halteelement 9 am zweiten Ende 13a des Ansatzstücks 13 mit dem uterusseitigen terminalen Abschnitt der Zervix Z in Eingriff. Das zweite Halteelement 9 steht zusätzlich zu oder in Alternative zu der ersten Halteeinrichtung 2 mit diesem terminalen Endabschnitt der Zervix Z in Eingriff Besonders bevorzugt ist das zweite Halteelement 9 ein ringförmig um das zweite Ende 13a des Ansatzstücks 13 aufblasbarer Ballon 16, der nicht nur mit dem terminalen uterusseitigen Abschnitt der Zervix Z in Eingriff tritt, bzw. die Übertragung einer Zugkraft auf die Zervix Z durch Bewegen des Führungselements 1 in Richtung vom Uterus U weg erlaubt (in Figur 6 nach links), sondern dichtet überdies die Zervix Z gegen den Uterus U ab, sodass ein Aufblasen des Uterus U mittels Mediums, das durch die Leitung 14 eingeblasen wird, möglich wird. Eine Leitung 14 ist entsprechend vorzugsweise längs des Führungselements 1 und des Ansatzstücks 13 angeordnet und endet in einem Abschnitt des Ansatzstücks 13, der auf der dem zweiten Ende 13a des Ansatzstücks 13 zugewandten Seite des zweiten Halteelements 9 liegt. Anstelle einer Saugglocke kann das erste Halteelement 9 auch durch zwei oder mehr Saugglocken gebildet sein, z.B. becher- oder ringnapfförmige Glocken.

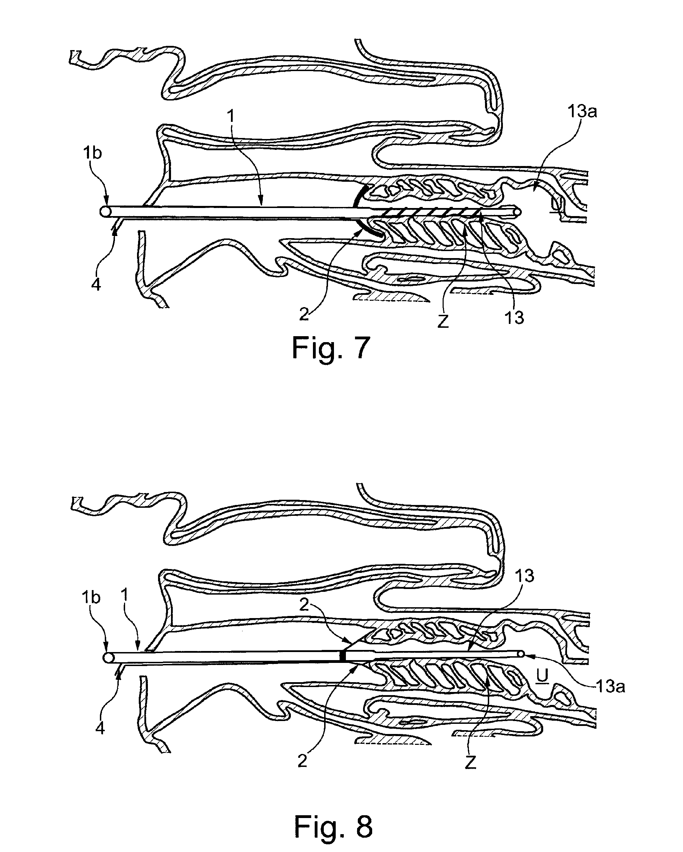

Figur 7 zeigt eine weitere Ausführungsform der Vorrichtung, in der das Ansatzstück 13 ein Außengewinde aufweist, mit dem das Ansatzstück 13 leichter durch Rotation durch die Zervix Z passieren kann. Wahlweise kann das Ansatzstück 13 an seinem zweiten Ende 13a eine Verdickung aufweisen, die vorzugsweise mindestens der Höhe des Außengewindes entspricht.

Figur 8 zeigt die Vorrichtung in einer Ausführung, in der das erste Halteelement 2 als mindestens zwei Zangengreifer ausgebildet ist, die durch Betätigen der Betätigungseinrichtung (nicht gezeigt), die z.B. im Bereich des ersten Endes 1b des Führungselements 1 eine Steuerungseinrichtung aufweist und mit einem terminalen Abschnitt der Zervix Z in Eingriff tritt.
- Bezugszeichenliste:: 9 zweites Halteelement
- 1 Führungselement: 10 zweite flexible Leitung
- 1a zweites Ende des Führungselements: 11 Sensorfläche
- 1b erstes Ende des Führungselements: 12 Mantel
- 2 erstes Halteelement: 13 Ansatzstück
- 3 flexible Leitung: 13a zweites Ende des Ansatzstücks
- 3a distales Ende der flexiblen Leitung: 13b erstes Ende des Ansatzstücks
- 3b proximales Ende der flexiblen Leitung: 14 Leitung
- 4 erste Betätigungseinrichtung: 15 zweite Betätigungseinrichtung
- 5 Abstandshalter bzw. Dichtung: 16 Ballon
- 6 Schiebeelement: U Uterus
- 7 Mandrin: Z Zervix
- 8 Führungseinrichtung: E Eileiter

## Patentansprüche

1. Kathetervorrichtung mit einer ersten-flexiblen Leitung (3), die längsverschieblich in einem Führungselement (1) angeordnet ist, das ein erstes Ende (1b) und ein dem ersten Ende (1b) gegenüberliegendes zweites Ende (1a) aufweist, wobei entlang der ersten flexiblen Leitung (3) eine Detektionseinrichtung angeordnet ist, deren Sensorfläche (11) in einem distalen Abschnitt der ersten flexiblen Leitung (3) gegenüber deren proximalen Ende (3b) liegt, welches am ersten Ende (1b) des Führungselements (1) angeordnet ist, und die erste flexible Leitung (3) eine zweite flexible Leitung (10) längsverschieblich enthält, an deren erstes Ende, das am proximalen Ende (3b) der ersten flexiblen Leitung (3) angeordnet ist, ein Behälter anschließbar ist und deren gegenüberliegendes zweites Ende über das distale Ende (3a) am distalen Abschnitt der ersten flexiblen Leitung (3) hinaus verschieblich ist, **dadurch gekennzeichnet, dass** die erste und die zweite flexible Leitung (3, 10) längs verschieblich in einem Mantel (12) angeordnet sind, der längsverschieblich im Führungselement (1) angeordnet ist, dass die Sensorfläche (11) am zweiten Ende des Mantels (12) angeordnet ist, das dessen am ersten Ende des Führungselements (1) angeordneten erstem Ende gegenüber liegt.

2. Kathetervorrichtung nach Anspruch 1, **dadurch gekennzeichnet, dass** der am zweiten Ende angeordnete Endabschnitt der zweiten flexiblen Leitung (10) aus der ersten flexiblen Leitung (3) vorschiebbar und bogenförmig gekrümmt ist, wobei der diesen Endabschnitt der zweiten flexiblen Leitung (10) umgebende Endabschnitt der ersten flexiblen Leitung (10) eine höhere Steifigkeit als die zweite flexible Leitung (10) aufweist.

3. Kathetervorrichtung nach einem der voranstehenden Ansprüche, **dadurch gekennzeichnet, dass** am zweiten Ende (1a) des Führungselements (1) ein erstes Halteelement (2) angeordnet ist, das mit einem terminalen Abschnitt der Zervix (Z) eines weiblichen Säugetiers in Eingriff bringbar ist,

4. Kathetervorrichtung nach einem der voranstehenden Ansprüche, **dadurch gekennzeichnet, dass** ein Ansatzstück (13) längsverschieblich im Führungselement (1) angeordnet ist oder ein Ansatzstück (13) mit seinem ersten Ende (13b) am zweiten Ende (1a) des Führungselements (1) angeordnet ist und das erste Ende (13b) des Ansatzstücks (13) über das erste Ende (1b) des Führungselements (1) hinaus verschieblich ist und/oder darüber hinausragt, wobei die erste flexible Leitung (3) längsverschieblich innerhalb des Ansatzstücks (13) anordnbar ist und die erste flexible Leitung (3) aus dem dem ersten Ende (13b) gegenüberliegenden zweiten Ende (13a) des Ansatzstücks (13) austritt.

5. Kathetervorrichtung nach einem der voranstehenden Ansprüche, **dadurch gekennzeichnet, dass** das erste Halteelement (2) eine Saugglocke, Saugöffnungen, einen Zangengreifer und/oder einen aufblasbaren Ballon aufweist oder daraus besteht, wobei das erste Halteelement (2) mit einer ersten Betätigungseinrichtung (4) gekoppelt ist, die ein am ersten Ende (1b) des Führungselements (1) angeordnetes Bedienelement aufweist und das erste Halteelement (2) mittels der Betätigungseinrichtung in einen ersten Zustand bringbar ist, in welchem das erste Halteelement (2) nicht mit der Zervix (Z) in Eingriff steht, und in einen zweiten Zustand, in welchem es mit einem der voneinander beabstandeten terminalen Abschnitte der Zervix in Eingriff steht.

6. Kathetervorrichtung nach einem der Ansprüche 2 bis 5, **dadurch gekennzeichnet, dass** innerhalb des Ansatzstücks (13) ein längsverschiebliches Schiebeelement (6) angeordnet ist, dessen zweites Ende (6a) seinem ersten Ende (6b) gegenüberliegt und ein Mandrin (7) und/oder einen aufblasbaren Ballon trägt, der über das zweite Ende (13a) des Ansatzstücks (13) ragt.

7. Kathetervorrichtung nach einem der voranstehenden Ansprüche, **dadurch gekennzeichnet, dass** innerhalb der ersten flexiblen Leitung (3) ein Schiebeelement angeordnet ist, das einen im Bereich des distalen Endes (3a) der ersten flexiblen Leitung (3) angeordneten Mandrin oder aufblasbaren Ballon trägt.

8. Kathetervorrichtung nach einem der voranstehenden Ansprüche, **dadurch gekennzeichnet, dass** entlang der ersten flexiblen Leitung (3) ein längs der ersten flexiblen Leitung (3) verschieblicher Führungsdraht angeordnet ist.

9. Kathetervorrichtung nach einem der voranstehenden Ansprüche, **dadurch gekennzeichnet, dass** das Führungselement (1) eine Abdichtungseinrichtung und eine längs des Führungselements angeordnete Leitung zur Insufflierung von Gas in den Uterus aufweist, deren Öffnung in einem Bereich zwischen der Abdichtungseinrichtung und dem zweiten Ende des Führungselements (1) oder dem zweiten Ende eines am Führungselement angeordneten Ansatzstücks (13) angeordnet ist.

10. Kathetervorrichtung nach einem der voranstehenden Ansprüche, **dadurch gekennzeichnet, dass** die zweite flexible Leitung (3, 10) an ihrem zweiten Ende einen Endabschnitt aufweist, der zu einer Spitze ausgezogen ist und der aus dem distalen Ende der ersten flexiblen Leitung herausbewegt werden kann, wobei der zu einer Spitze ausgezogene Endabschnitt einen Außendurchmesser von 0,3 bis 0,7 mm und einen Innendurchmesser von 0,1 bis 0,4 mm bei einer Länge von 3 bis 8 cm hat.

11. Kathetervorrichtung nach einem der voranstehenden Ansprüche, **dadurch gekennzeichnet, dass** die erste flexible Leitung (3) einen Außendurchmesser von 1,4 bis 2,2 mm auf weist, einen Innendurchmesser von 1,0 bis 2,0 mm und eine Elastizität, die bei einer freien Länge von 50 mm eine maximale axiale Widerstandskraft gegen das Knicken von 2500 mN aufweist, und die zweite flexible Leitung (10) einen zu einer Spitze ausgezogenen Endabschnitt mit einem Außendurchmesser von 0,3 bis 0,7 mm und einem Innendurchmesser von 0,1 bis 0,4 mm mit einer Elastizität, die bei einer freien Länge von 10 mm eine maximale axiale Widerstandskraft von 1000 mN aufweist.

12. Kathetervorrichtung nach einem der voranstehenden Ansprüche, **dadurch gekennzeichnet, dass** die erste flexible Leitung (3) eine Elastizität hat, die bei einer freien Länge von 50 mm eine axiale Widerstandskraft von 1900 bis 2100 mN gegen das Knicken aufweist.

13. Kathetervorrichtung nach einem der Ansprüche 10 bis 12, **dadurch gekennzeichnet, dass** der zu einer Spitze ausgezogene Endabschnitt eine Flexibilität mit einer Widerstandskraft bei einer freien Länge von 10 mm von 500 bis 1000 mN aufweist und/oder bei einer freien Länge von 17,5 mm von 80 bis 240 mN und/oder bei einer freien Länge von 35 mm von 20 bis 80 mN aufweist.

14. Kathetervorrichtung nach einem der Ansprüche 10 bis 13, **dadurch gekennzeichnet, dass** die zweite flexible Leitung angrenzend an den zu einer Spitze ausgezogenen Endabschnitt einen Außendurchmesser von 0,8 bis 1,8 mm und einen Innendurchmesser von 0,2 bis 1,4 mm aufweist und dieselbe oder eine höhere Flexibilität.

## Claims

1. Catheter device having a first flexible conduit (3) which is longitudinally slidable in a guiding element (1) having a first end (1b) and a second end (1a) opposite the first end (1b), wherein a detection device is arranged along the first flexible conduit (3), the sensor surface (11) of which lies in a distal section of the first flexible conduit (3) opposite its proximal end (3b), which is arranged at the first end (1b) of the guiding element (1), and the first flexible conduit (3) comprising a second flexible conduit (10) longitudinally slidably, to the first end of which, arranged at the proximal end (3b) of the first flexible conduit (3), a container is connectable and the opposite second end of which is slidably beyond the distal end (3a) at the distal section of the first flexible conduit (3), **characterized in that** the first and the second flexible conduit (3, 10) are arranged longitudinally slidable in a sheath (12) that is arranged longitudinally slidable in the guiding element (1) such that the sensor surface (11) is arranged at the second end of the sheath (12) which is opposite of the first end thereof that is arranged at the first end of the guiding element (1).

2. Catheter device according to claim 1, **characterized in that** the terminal section of the second flexible conduit (10) arranged at the second end can be advanced out of the first flexible conduit (3) and is arcuately bent, wherein the terminal section of the first flexible conduit (10) is surrounding this terminal section of the second flexible conduit (10) has a higher stiffness than the second flexible conduit (10).

3. Catheter device according to one of the preceding claims, **characterized in that** at the second end (1a) of the guiding element (1) first fixation element (2) is arranged which is engagable with a terminal section of the cervix (Z) of a female mammal.

4. Catheter device according to one of the preceding claims, **characterized in that** in the guiding element (1) an attachment piece of (13) is arranged longitudinally slidable, or an attachment piece (13) is arranged with its first end (13b) at the second end (1a) of the guiding element (1) and that the first end (13b) of the attachment piece (13) can be advanced beyond the first end (1b) of the guiding element (1) and/or projects beyond that, wherein the first flexible conduit (3) can be arranged longitudinally slidable within the attachment piece (13) and the first flexible conduit (3) projects from the second end (13a) of the attachment piece (13), the second end (13a) lying opposite the first end (13b).

5. Catheter device according to one of the preceding claims, **characterized in that** the first fixation element (2) comprises or consists of a suction cone, suction openings, a gripper finger and/or inflatable balloon, wherein the first fixation element (2) is coupled to a first operating device (4) having an operational control arranged at the first end (1b) of the guiding element (1), and the first fixation element (2) by means of the operational control can be brought into a first status in which the first fixation element (2) is not engaged with the cervix (Z), and into a second status, in which it is engaged of one to the terminal sections of the cervix which are spaced from one another.

6. Catheter device according to one of claims 2 to 5, **characterized in that** within the attachment piece (13) a longitudinally slidably guiding element (6) is arranged, the second end (6a) of which is opposite its first end (6b) and carries a mandrin (7) and/or an inflatable balloon that projects beyond the second end (13a) of the attachment piece (13).

7. Catheter device according to one of the preceding claims, **characterized in that** within the first flexible conduit (3) a sliding element is arranged that carries a mandarin (7) or inflatable balloon arranged in the region of the distal end (3a) of the first flexible conduit (3).

8. Catheter device according to one of the preceding claims, **characterized in that** along the first flexible conduit (3) a guiding wire slidable along the first flexible conduit (3) is arranged.

9. Catheter device according to one of the preceding claims, **characterized in that** the guiding element (1) as a sealing device and a conduit arranged along the guiding element for insufflation of gas into the uterus, the opening of the conduit being arranged in a region between the sealing device and the second end of the guiding element (1) or the second end of an attachment piece arranged at the guiding element.

10. Catheter device according to one of the preceding claims, **characterized in that** the second flexible conduit (3), (10) its second end has a terminal section which is pulled out to a tip and which can be moved out of the distal end of the first flexible conduit, wherein the terminal section pulled out to a tip has an outer diameter of 0.3 to 0.7 mm and an inner diameter of 0.1 to 0.4 mm at a length of 3 to 8 cm.

11. Catheter device according to one of the preceding claims, **characterized in that** the first flexible conduit (3) has an outer diameter of 1.4 to 2.2 mm, and an inner diameter of 1.0 to 2.0 mm and an elasticity which at a free length of 50 mm has a maximal axial resistance against buckling of 2500 mN and that the second flexible conduit (10) has a terminal section pulled out to a tip having an outer diameter of 0.3 to 0.7 mm and an inner diameter 0.1 to 0.4 mm having an elasticity which at a free length of 10 mm has a maximum axial resistance of 1000 mN.

12. Catheter device according to one of the preceding claims, **characterized in that** the first flexible conduit has an elasticity which at a free length of 50 mm has an axial resistance of 1900 to 2100 mN against buckling.

13. Catheter device according to one of claims 10 to 12, **characterized in that** the terminal section pulled out to a tip has a flexibility with a resistance at a free length of 10 mm of 500 to 1000 mN and/or at a free length of 17.5 mm of 80 to 240 mN and/or at a free length of 35 mm of 20 to 80 mN.

14. Catheter device according to one of claims 10 to 13, **characterized in that** the second flexible conduit adjacent the terminal section pulled out to a tip has an outer diameter of 0.8 to 1.8 mm and an inner diameter of 0.2 to 1.4 mm and the same or a higher flexibility.

## Revendications

1. Dispositif cathéter comportant un premier conduit flexible (3), qui est mobile dans la sens de la longueur dans un élément de guidage (1), élément présentant une première extrémité (1b) et une seconde extrémité (1a) opposée à la première extrémité (1b), où un dispositif de détection est disposé le long du premier conduit flexible (3), dispositif dont la surface de capteur (11) est situé dans une section distale du premier conduit flexible (3) par rapport à son extrémité proximale (3b) qui est agencée au niveau de la première extrémité (1b) de l'élément de guidage (1) et le premier conduit flexible (3) contient mobile dans la sens de la longueur un second conduit flexible (10), un contenant peut être connecté à la première extrémité qui est située à l'extrémité proximale (3b) du premier conduit flexible (3), et dont la seconde extrémité opposée est mobile au-dessus et au-delà de l'extrémité distale (3a) au niveau de la section distale du premier conduit flexible (3),
**caractérisé en ce que** le premier conduit flexible et le second conduit flexible (3, 10) sont disposés mobiles dans la sens de la longueur dans une enveloppe (12) qui est aménagée mobile dans la sens de la longueur dans l'élément de guidage (1), que la surface de capteur (11) est disposée au niveau de la seconde extrémité de l'enveloppe (12), la première extrémité de celle-ci est opposée à la première extrémité de l'élément de guidage (1).

2. Dispositif cathéter selon la revendication 1, **caractérisé en ce que** la section d'extrémité du second conduit flexible (10) disposée au niveau de la seconde extrémité peut s'extraire du premier conduit flexible (3) et est recourbée en arc-de-cercle, où la section d'extrémité du premier conduit flexible (10) entourant ladite section d'extrémité du second conduit flexible (10) présente une plus grande rigidité que le second conduit flexible (10).

3. Dispositif cathéter selon l'une des revendications précédentes, **caractérisé en ce qu'**un premier élément de retenue (2) est disposé au niveau de la seconde extrémité (1a) de l'élément de guidage, que l'on peut mettre en prise avec une section terminale du cervix (Z) d'un mammifère.

4. Dispositif cathéter selon l'une des revendications précédentes, **caractérisé en ce qu'**un embout (13) est agencé mobile dans la sens de la longueur dans l'élément de guidage (1) ou un embout (13) est agencé par sa première extrémité (13b) au niveau de la seconde extrémité (1a) de l'élément de guidage (1) et la première extrémité (13b) de l'embout (13) est mobile au-dessus et au-delà de la première extrémité (1b) de l'élément de guidage et/ou dépasse de celle-ci, où le premier conduit flexible (3) peut être agencé mobile dans la sens de la longueur à l'intérieur de l'embout (13) et le premier conduit flexible (3) sort de la seconde extrémité (13a) de l'embout (13), extrémité opposée à la première extrémité (13b).

5. Dispositif cathéter selon l'une des revendications précédentes, **caractérisé en ce que** le premier élément de retenue (2) contient une cloche d'aspiration, des ouvertures d'aspiration, un preneur à pinces et/ou un ballon gonflable ou se compose de ces derniers, où le premier élément de retenue (2) est couplé à un premier système de commande (4) qui présente un élément de commande agencé au niveau de la première extrémité (1b) de l'élément de guidage (1) et le premier élément de retenue (2) peut adopter un premier état à l'aide du système de commande dans lequel le premier élément de retenue (2) n'est pas engrené avec le cervix (Z) et un second état, dans lequel l'élément de retenue est engrené avec l'une des sections terminales de du cervix espacées l'une de l'autre.

6. Dispositif cathéter selon l'une des revendications 2 à 5, **caractérisé en ce qu'**un élément coulissant (6) mobile dans le sens de la longueur est agencé à l'intérieur de l'embout (13), élément dont la seconde extrémité (6a) est opposée à sa première extrémité (6b) et porte un mandrin (7) et/ou un ballon gonflable qui dépasse de la seconde extrémité (13a) de l'embout (13).

7. Dispositif cathéter selon l'une des revendications précédentes, **caractérisé en ce qu'**un élément coulissant est agencé à l'intérieur du premier conduit flexible (3), qui porte un ballon gonflable ou bien un mandrin agencé dans la zone de l'extrémité distale (3a) du premier conduit flexible (3).

8. Dispositif cathéter selon l'une des revendications précédentes, **caractérisé en ce qu'**un fil de guidage, mobile le long du premier conduit flexible (3) longe le premier conduit flexible (3).

9. Dispositif cathéter selon l'une des revendications précédentes, **caractérisé en ce que** l'élément de guidage (1) contient un dispositif d'étanchéité et un conduit agencé le long de l'élément de guidage pour insuffler du gaz dans l'utérus, dont l'ouverture est prévue dans une zone comprise entre le dispositif d'étanchéité et la seconde extrémité de l'élément de guidage (1) ou bien la seconde extrémité d'un embout (13) agencé au niveau de l'élément de guidage.

10. Dispositif cathéter selon l'une des revendications précédentes, **caractérisé en ce que** le second conduit flexible (3, 10) présente une section d'extrémité au niveau de sa seconde extrémité, section étirée en pointe, et que l'on peut extraire de l'extrémité distale du premier conduit flexible, où la section d'extrémité étirée en pointe présente un diamètre externe de 0,3 à 0,7 mm et un diamètre interne de 0,1 à 0,4 mm pour une longueur de 3 à 8 cm.

11. Dispositif cathéter selon l'une des revendications précédentes, **caractérisé en ce que** le premier conduit flexible (3) présente un diamètre externe de 1,4 à 2,2 mm, un diamètre interne de 1,0 à 2,0 mm et une élasticité ayant une force de résistance axiale maximale au flambage de 2500 mN pour une longueur libre de 50 mm, et le second conduit flexible (10) comporte une section d'extrémité étirée en pointe avec un diamètre externe de 0,3 à 0,7 mm et un diamètre interne de 0,1 à 0,4 mm, avec une élasticité d'une force de résistance axiale maximale de 1000 mN pour une longueur libre de 10 mm.

12. Dispositif cathéter selon l'une des revendications précédentes, **caractérisé en ce que** le premier conduit flexible (3) a une élasticité d'une force de résistance axiale maximale au flambage de 1900 à 2100 mN pour une longueur libre de 50 mm,

13. Dispositif cathéter selon l'une des revendications 10 à 12, **caractérisé en ce que** la section d'extrémité étirée en pointe a une flexibilité d'une force de résistance de 500 à 1000 mN pour une longueur libre de 10 mm et/ou de 80 à 240 mN pour une longueur libre de 17,5 mm et/ou de 20 à 80 mN pour une longueur libre de 35 mm.

14. Dispositif cathéter selon l'une des revendications 10 à 13, **caractérisé en ce que** le second conduit flexible comporte une section d'extrémité étirée en pointe et limitrophe d'un diamètre externe de 0,8 à 1,8 mm et un diamètre interne de 0,2 à 1,4 mm, et la même flexibilité ou bien une flexibilité accrue.
